# EUROPEAN PATENT APPLICATION

(11) **EP 1 346 723 A1**
(43) Date of publication of application: **24.09.2003**
(21) Application number: 03005037.1
(22) Date of filing: 06.03.2003
(51) Int. Cl.: A61K 31/70, A61K 9/50

(54) **Multiparticulate enteric coated and multiparticulate enteric coated prolonged release formulations containing NADH**

(30) Priority: 21.03.2002 IT BO20020140
(71) Applicant: Valpharma S.A., 47899 Serravalle (SM)
(72) Inventor: Valducci, Roberto, 47039 Savignano sul Rubicone (FC) (IT); Alighieri, Tiziano, 47900 Rimini (IT); Avanessian, Serozh, 47900 Rimini (IT)
(74) Representative: Negrini, Elena

(57) **Abstract**

Formulations for the oral administration of NADH in multiparticulate enteric coated form and/or dosed either into gelatine capsules or sachets or dispensers.

## Description

This invention refers to the use of NADH as food supplement.

Nicotinamide-adenine-dinucleotide in reduced form (NADH) has antioxidant properties and performs a vital role in the processes of energy production.

Various publications in the USA and Europe refer to said molecule positive effects like the increase of energy, the protection of cells from damages caused by free radicals and the potentiation of the immunitary system.

The biochemical action of NADH, hydrogenated reduced form of NAD (nicotinamide-adenine-dinucleotide), is well-known: it is the coenzyme or active form of vitamin B₃, which activates various enzymes and is necessary to oxidize foodstuffs.

In the process of cell respiration (Krebs cycle), which has the aim to obtain energy from the catabolism of sugars, lipids and amino acids, NADH is involved in the production of adenosine triphosphatase (ATP), i.e. the substance that runs all the energy-requiring-reactions of the cells. NAD can be either synthesized from the living cells (with ATP expenditure), or introduced with the diet: in fact it is present in many proteinic foods and, in minor measure, in vegetables.

It has been suggested that the integration of the diet with a NADH-based product can increase the resistance to the physical effort: hence the definition of "pro-energetic" of products on the market.

Although NADH can be integrated with diet, it is anyway a relatively instable compound, sensitive to the acid of the stomach and, therefore, the oral integration gives unsteady and less significant results.

NADH and NADPH (derivative phosphate of analogous biological value) are instable to the pH values of the gastric environment.

Some methods have been proposed (see USA patent n° US. 5,332,727) for the manufacture of coated tablets, which protect the NADH content in the stomach: several ingredients are cited for the enteric coating, together with an example of a tablet preparation containing a stabilizing agent and a "filling" agent, taken from the established pharmaceutical technique.

The above mentioned patent reports an example for the manufacture of a tablet dosage form and refers the possibility to coat tablet or capsule dosage forms with an external layer to give protection toward the acid environment.

All the preparations mentioned in the referred patent are "monolithic", i.e. only one externally coated unit in tablet or capsule dosage form has to guarantee both the protection in acid environment and the suitable dispersion of NADH in the intestinal environment to assure the absorption.

The literature describes preparations for the oral administration in "multiparticulate" form, i.e. micro-particles usually with a diameter of about 1 mm and with almost spherical form, each one individually constituted to guarantee both the protection in acid environment and the content release according to the required specification.

Further advantages of the multiparticulate systems are the improved kinetic characteristics of said micro-particles which, particularly if with a diameter smaller than 2 mm, behave in the organism like "liquid" components, with uniform intestinal transit times and a better dispersion in the alimentary tract with the consequent optimization of the absorption (ref.: *"Multiparticulate oral dosage forms: technology and biopharmaceutics*" edited by C.D. Melia, N. Washington, C.G. Wilson).

The uniformity in the transit times is specially significant if we take into consideration that, in particular cases, the "monolithic" forms may remain in the stomach for long periods: in such a case there is always the risk to partly or completely lose the content of the active ingredient. The multiparticulate forms assure shorter times of gastric retention.

The object of this invention is the manufacture of formulations suitable for the oral administration, containing NADH or NADPH, in multiparticulate enteric coated form or multiparticulate enteric coated prolonged release form.

Hereafter are described methods for the manufacture and the in-vitro testing of multiparticulates, pellets or microgranules, having enteric coated or enteric coated prolonged release profile, ready to be dosed either into capsules or sachets or suitable dispensers.

Said methods provide for the layering of NADH on inert cores, eventually with binding excipients like polyvinylpyrrolidone or polyethyleneglycol or derivatives, stabilizers like tocopherol and/or ascorbic palmitate, and other excipients taken from the established pharmaceutical techniques.

The cores containing NADH can be prepared by extrusion and / or spheronization processes, by using binders like polyvinylpyrrolidone or polyethyleneglycol or derivatives, plasticizers like microcrystalline cellulose or starch or derivatives, or other excipients derived from the common pharmaceutical techniques.

The cores of this invention can be coated with enteric coating agents, like hydroxypropylmethylcellulosephtalate, celluloseacetatephtalate, hydroxypropylmethylcellulosesuccinate, polymers of acrylic and metacrylic acid, other derivatives from the pregelatinised acetylated starch cellulose, shellac, polyvinylpyrrolidone, eventually charged with plasticizer agents and/or colorants and every mixture in various proportions of the same.

The formulations of this invention can also be coated with substances suitable to modify the NADH release, like polymers of acrylic and metacrylic acid, ethylcellulose or other cellulose derivatives, shellac, paraffin, fat acids or mixtures in any proportion of said components, eventually charged with plasticizer agents and/or colorants subsequently coated to guarantee the enteric coating.

The optimization of the pharmacokinetic parameters gained by means of the multiparticulate systems, thanks to the optimization of the transit times and the approved absorption, permits to reach suitable regimens as food supplement or for therapeutic treatment, like the suggested utilization of NADH in patients with Parkinson's disease.

The invention is explained by the following examples.

### Example n° 1 - Composition:

| | |
|---|---|
| β-NADH | 45 g |
| PVP 40% in purified water | 300 g |
| Ethanol | 1800 g |
| Vitamin E | 90 g |
| Microcrystalline cellulose | 2100 g |
| Maize starch | 900 g |
| Tot. | 5145 g |

Cellulose and maize starch, placed in a jacketed Z Lleal granulator, type AM5, were mixed for 30 minutes. Then a suspension containing β-NADH, PVP, ethanol and vitamin E was prepared by means of a homogenizer Silverson, L4R-model. Such suspension was utilized to wet the mixture of powders in the Z-granulator.

At a temperature of 40°C, a mass of adequate consistency was obtained through granulation and then extruded utilizing a Fuji-Paudal extruder, DomeGran-model.

The so obtained compound was spheronized in a Fuji-Paudal spheronizator, Q230T-model. The obtained multiparticulate was then desiccated in a drier at 40°C for 24 hours and sieved with nets of 500 and 1200 microns.

### Example n° 1.1 - Enteric coated formulation

1 Kg of spheroids obtained as described in example 1, placed in a Glatt fluid bed, Uniglatt model, was coated with the following solution:

| | |
|---|---|
| HPMCP55 5% in acetone-ethanol | 1000 g |

After desiccation in a drier at 35°C for 14 hours, the spheroids were encapsulated by dosing 5 mg / capsule of β-NADH, utilizing a Bonapace capsule machine, In-Cap model.

The so obtained capsules were tested to evaluate the in-vitro dissolution, thus obtaining the following results:

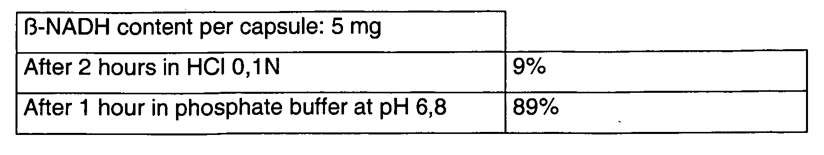

### Example 1.2 - Enteric coated formulation

1 Kg of spheroids obtained as described in example 1, put in a Glatt fluid bed, Uniglatt model, was coated with the following solution:

| | |
|---|---|
| CAP 5% in acetone-ethanol | 1000 g |
| Acetylated monoglycerides | 2,5 g |

After desiccation of the multiparticulate in a drier at 35°C for 14 hours, the spheroids were encapsulated by dosing 5 mg / capsule of β-NADH, utilizing a Bonapace capsule machine, In-Cap model. The so obtained capsules were tested to evaluate the in-vitro dissolution, thus obtaining the following results:

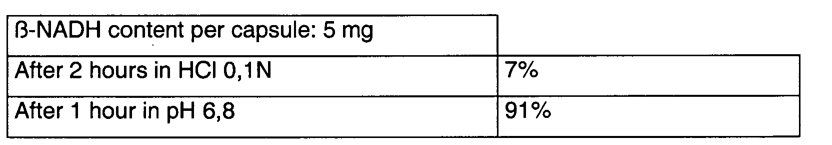

### Example n° 2 - Composition:

| | |
|---|---|
| PVP 40% in purified water | 300 g |
| Ethanol | 1800 g |
| β-NADH | 45 g |
| Microcrystalline cellulose | 1800 g |
| Maize starch | 900 g |
| Tot. | 4845 g |

In a jacketed Z Lleal granulator, AM5 model, cellulose, maize starch and β-NADH were mixed for 30 minutes. A solution of PVP and ethanol was prepared with a pneumatic mixer. Such solution was utilized to wet the mixture of powders in the Z-granulator.

At a temperature of 40°C, the compound was kneaded up to the obtainment of a mass of adequate consistency, which was extruded by means of an extruder Fuji-Paudal, DomeGran model.

The so obtained compound was spheronized with Fuji-Paudal, Q230T model.

The obtained multiparticulate, desiccated in a drier at 40°C for 24 hours, was subsequently sieved with nets of 500 and 1200 microns.

### Example n° 2.1 - Enteric coated prolonged release formulation

1 Kg of spheroids obtained as described in example 2 was put in a Glatt fluid bed, Uniglatt model, and was coated with the following solution:

| | |
|---|---|
| Eudragit RS 10% in acetone | 540 g |
| Eudragit RL 10% in acetone | 60 g |
| Ethanol | 300 g |
| Tot. | 900 g |

and then with:

| | |
|---|---|
| HPMCP55 5% in acetone-ethanol | 600 g |

After desiccation in a drier at 35°C for 14 hours, the multiparticulate was encapsulated by dosing the spheroids in a quantity equivalent to 5 mg / capsule of β-NADH, utilizing a Bonapace capsule machine, In-Cap model.

The so obtained capsules were tested to evaluate the in-vitro dissolution, thus obtaining the following results:

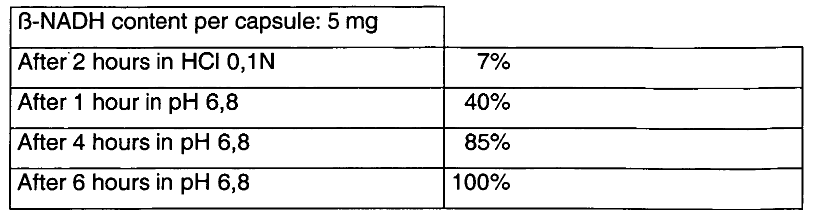

### Example n° 2.2 - Enteric coated prolonged release formulation

1 Kg of spheroids obtained as described in example 2 was put in a Glatt fluid bed, Uniglatt model, and coated with the following solution:

| | |
|---|---|
| Ethocel 5% in acetone | 400 g |
| CAP 5% in acetone-ethanol | 500 g |
| Ethanol | 300 g |
| Tot. | 1200 g |

After desiccation of the spheroids in a drier at 35°C for 14 hours, the spheroids themselves were encapsulated by dosing a quantity equivalent to 5 mg / capsule of β-NADH, utilizing a Bonapace capsule machine, In-Cap model.

The so obtained capsules were tested to evaluate the in-vitro dissolution, thus obtaining the following results:

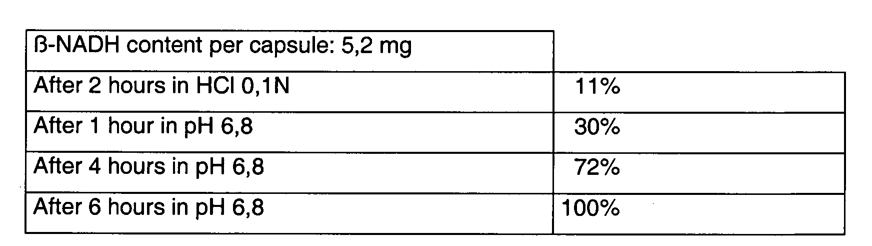

### Example n° 3 - Composition:

| | |
|---|---|
| β-NADH | 15 g |
| PVP 40% in purified water | 100 g |
| Purified water | 500 g |
| Sodium bicarbonate | 50 g |
| Microcrystalline cellulose | 700 g |
| Maize starch | 300 g |
| Tot. | 1665 g |

Cellulose and maize starch were put in a jacketed Z Lleal granulator, AM5 model, and mixed for 30 minutes.

A suspension with β-NADH, PVP, water and sodium bicarbonate, was prepared with a Silverson homogenizer, L4R model. Such suspension was used to wet the mixture of powders in the Z-granulator. With a temperature of 40°C, the obtained compound was kneaded up to the obtainment of a mass of adequate consistency, which was granulated with a Kahl granulator, mod. 14-175.

The compound was spheronized with spheronizator Fuji-Paudal, Q230T model. The obtained spheroids, desiccated at 40°C for 24 hours, were sieved with nets of 500 and 1200 microns.

### Example n° 3.1 - Enteric coated formulation

0,5 Kg of spheroids obtained as described in example 3 were put in a Glatt fluid bed, Uniglatt model, and coated with the following solution:

| | |
|---|---|
| HPMCP55 5% in acetone-ethanol | 500 g |

After desiccation in a drier at 35°C for 14 hours, the spheroids were encapsulated by dosing a quantity equivalent to 5 mg / capsule of β-NADH, utilizing a Bonapace capsule machine, In-Cap model. The so obtained capsules were tested to evaluate the in-vitro dissolution, thus obtaining the following results:

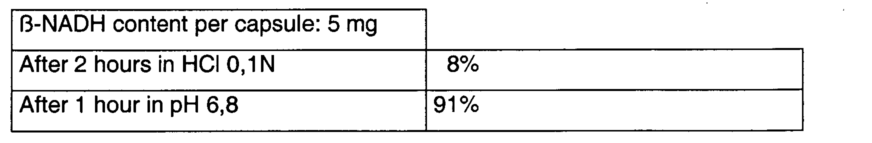

### Example n° 4 - Composition:

| | |
|---|---|
| β-NADH | 15 g |
| Maize starch | 150 g |
| Sugar spheres (size 20) | 850 g |
| PVP 40% in purified water | 40 g |
| Purified water | 40 g |
| Tot. | 1031 g |

The neutral cores were put in a rotating pan and sprayed with a solution of PVP and water; then the mixture of maize starch and β-NADH was applied; the so obtained compound was desiccated in a drier at 35°C for 14 hours.

### Example n° 4.1 - Enteric coated formulation

0,8 Kg of spheroids obtained as described in example 4 were coated in a fluid bed, Uniglatt model, with the following solution:

| | |
|---|---|
| Eudragit L 30D | 100 g |
| Purified water | 300 g |
| Tot. | 400 g |

After desiccation in a drier at 35°C for 14 hours, the spheroids were encapsulated by dosing a quantity equivalent to 5 mg / capsule of β-NADH, utilizing a Bonapace capsule machine, In-Cap model.

The obtained capsules were tested to evaluate the in-vitro dissolution, thus obtaining the following results:

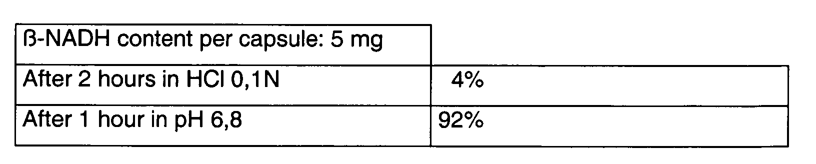

### Example n° 5 - Composition:

| | |
|---|---|
| β-NADH | 45 g |
| Sugar spheres (size 18) | 3000 g |
| PVP 40% in purified water | 120 g |
| Purified water | 600 g |
| Sodium bicarbonate | 20 g |
| Tot. | 3093 g |

The sugar spheres were put in a Glatt fluid bed, GPCG3 model, equipped with a rotor system. A solution of β-NADH, PVP, water and sodium bicarbonate was prepared by means of a pneumatic mixer. Such solution was sprayed on the sugar spheres and the obtained compound was desiccated in a drier at 35°C for 14 hours.

### Example n° 5.1 Enteric coated prolonged release formulation

1 Kg of spheroids obtained as described in example 5 was put in a Glatt fluid bed, Uniglatt model, and coated with the following solution:

| | |
|---|---|
| Eudragit RS 10% in acetone | 360 g |
| Eudragit RL 10% in acetone | 40 g |
| Ethanol | 200 g |
| Tot. | 600 g |

and then with:

| | |
|---|---|
| HPMCP55 5% in acetone-ethanol | 700 g |

After desiccation in a drier at 35°C for 14 hours, the multiparticulate was encapsulated by dosing a quantity equivalent to 5 mg / capsule of β-NADH, utilizing a Bonapace capsule machine, In-Cap model.

The obtained capsules were tested to evaluate the in-vitro dissolution, thus obtaining the following results:

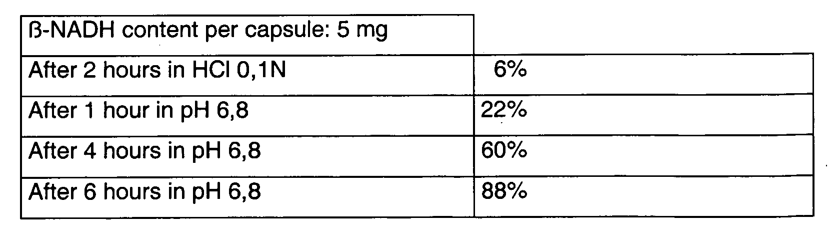

### Example n° 5.2 - Enteric coated prolonged release formulation

1 Kg of spheroids obtained as described in example 5 was put in a Glatt fluid bed, Uniglatt model, and coated with the following solution:

| | |
|---|---|
| Ethocel 5% in acetone | 300 g |
| Ethanol | 150 g |
| Tot. | 450 g |

and then with:

| | |
|---|---|
| Eudragit L 30D | 200 g |
| Purified water | 300 g |
| Tot. | 500 g |

After desiccation in a drier at 35°C for 14 hours, the spheroids were encapsulated by dosing 5 mg / capsule of β-NADH, utilizing a Bonapace capsule machine, In-Cap model. The obtained capsules were tested to evaluate the in-vitro dissolution, thus obtaining the following results:

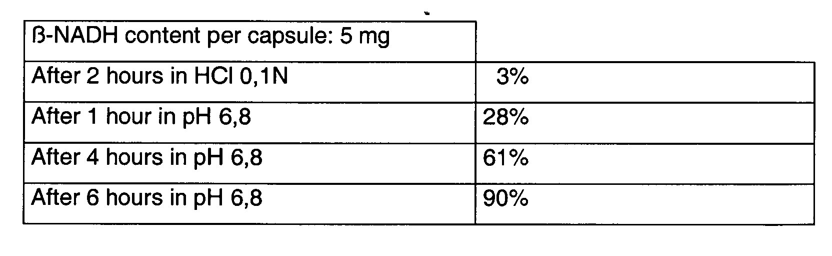

### Example n° 6 - Composition:

| | |
|---|---|
| β-NADH | 30 g |
| Sugar spheres (size 18) | 2000 g |
| PVP 40% in purified water | 80 g |
| Ethanol | 400 g |
| Tot. | 2062 g |

The neutral cores were put in rotating pan. A solution with β-NADH, PVP and Ethanol was prepared by a pneumatic mixer. Such solution was sprayed on the sugar spheres. The obtained compound was desiccated in a drier at 35°C for 14 hours.

### Example n° 6.1 - Enteric coated prolonged release formulation

1 Kg of spheroids obtained as described in example 6 was put in a rotating pan and coated with the following solution:

| | |
|---|---|
| Ethocel 5% in acetone-ethanol | 800 g |

and then with:

| | |
|---|---|
| PVP 20% in ethanol | 131 g |
| Shellac 40% in ethanol | 429 g |
| Acetylated monoglycerides 9 - 45 | 27 g |
| Acetone | 107 g |
| Ethanol | 106 g |
| Tot. | 800 g |

After desiccation of the spheroids in a drier at 35°C for 14 hours, such spheroids were encapsulated by dosing a quantity equivalent to 5 mg / capsules of β-NADH, utilizing a Bonapace capsule machine, In-Cap model. The so obtained capsules were tested to evaluate the in-vitro dissolution, thus obtaining the following results:

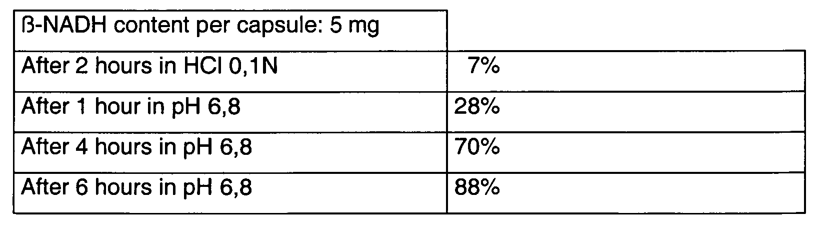

### Example n° 7 - Composition:

| | |
|---|---|
| β-NADH | 90 g |
| Microcrystalline cellulose | 2100 g |
| Maize starch | 900 g |
| PVP 40% in purified water | 400 g |
| Purified water | 1800 g |
| Tot. | 3250 g |

Cellulose and maize starch were put in a Glatt fluid bed, GPCG3 model, equipped with a rotor system.

A solution of β-NADH, PVP and water was prepared by means of a pneumatic mixer: such solution was sprayed on the mixture of powders.

The obtained spheroids were desiccated in a drier at 40°C for 24 hours and selected with nets of 500 and 1200 microns.

### Example n° 7.1 - Enteric coated prolonged release formulation

1 Kg of spheroids obtained as described in example 7 was put in a Glatt fluid bed, Uniglatt model, and coated with the following solution:

| | |
|---|---|
| Ethocel 5% in acetone | 300 g |
| Ethanol | 150 g |
| Tot. | 450 g |

and then with:

| | |
|---|---|
| HPMCP55 5% in acetone-ethanol | 700 g |

After desiccation in a drier at 35°C for 14 hours, the spheroids were encapsulated in a quantity equivalent to 5 and 10 mg / capsule of β-NADH, utilizing a Bonapace capsule machine, In-Cap model.

The so obtained capsules were tested to evaluate the in-vitro dissolution, thus obtaining the following results:

| | | |
|---|---|---|
| β-NADH mg content per capsule: | 5 | 10 |
| After 2 hours in HCI 0,1 N | 5% | |
| After 1 hour in pH 6,8 | 45% | |
| After 4 hours in pH 6,8 | 80% | |
| After 6 hours in pH 6,8 | 92% | |

### Example n° 8 - Composition:

| | |
|---|---|
| β-NADH | 250 g |
| Microcrystalline cellulose spheres | 8000 g |
| PVP 40% in purified water | 2200 g |
| Ethanol | 2500 g |
| Vitamin E | 500 g |
| Tot. | 13450 g |

The microcrystalline cellulose spheres were placed in a Glatt fluid bed, GPCG3 model, equipped with a rotor system; a solution with β-NADH, PVP, ethanol and vitamin E was prepared by means of a pneumatic mixer. Such solution was sprayed on the sugar spheres and the spheroids were desiccated in a drier at 35°C for 14 hours.

### Example 8.1 - Enteric coated formulation

1 Kg of spheroids obtained as described in example 8 was put in a Glatt fluid bed, Uniglatt model, and coated with the following solution:

| | |
|---|---|
| CAP 10% in aqueous solution | 1000 g |

After desiccation in a drier at 35°C for 14 hours, the spheroids were encapsulated by dosing 5 mg and 10 mg / capsule of β-NADH, utilizing a Bonapace capsule machine, In-Cap model. The obtained capsules were tested to evaluate the in-vitro dissolution, thus obtaining the following results:

| | | |
|---|---|---|
| β-NADH mg content per capsule: | 5 | 10 |
| After 2 hours in HCI 0,1 N | 6% | |
| After 1 hour in pH 6,8 | 92% | |

### Example n° 8.2 - Enteric coated prolonged release formulation

1 Kg of spheroids obtained as described in example 8 was put in a Glatt fluid bed, Uniglatt model, and was coated with the following solution:

| | |
|---|---|
| Ethocel 5% in acetone | 300 g |
| Ethanol | 150 g |
| Tot. | 450 g |

and then with:

| | |
|---|---|
| HPMCP55 5% in acetone-ethanol | 700 g |

After desiccation in a drier at 35°C for 14 hours, the spheroids were encapsulated in a quantity equivalent to 5 and 10 mg / capsule of β-NADH, utilizing a Bonapace capsule machine, In-Cap model. The obtained capsules were tested to evaluate the in-vitro dissolution, thus obtaining the following results:

| | | |
|---|---|---|
| β-NADH mg content per capsule: | 5 | 10 |
| After 2 hours in HCI 0,1 N | 6% | |
| After 1 hour in pH 6,8 | 35% | |
| After 4 hours in pH 6,8 | 59% | |
| After 6 hours in pH 6,8 | 89% | |

### Example n° 8.3 - Enteric coated formulation

0,7 Kg of spheroids obtained as described in example 8 were put in a Glatt fluid bed, Uniglatt model, and coated with the following solution:

| | |
|---|---|
| PVP 20% in ethanol | 49 g |
| Shellac 40% in ethanol | 161 g |
| Acetylated monoglycerides | 10 g |
| Acetone | 40 g |
| Ethanol | 40 g |
| Tot. | 300 g |

After desiccation in a drier at 35°C for 14 hours, the spheroids were encapsulated by dosing a quantity equal to 5 mg / capsule of β-NADH, utilizing a Bonapace capsule machine, In-Cap model. The obtained capsules were tested to evaluate the in-vitro dissolution, thus obtaining the following results:

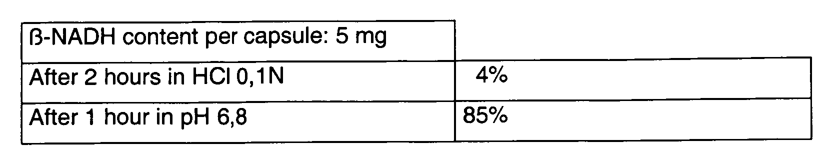

### Example n° 8.4 - Enteric Coated formulation

1 Kg of spheroids obtained as described in example 8 was put in a Glatt fluid bed, Uniglatt model, equipped with a rotor system, and was coated with the following solution:

| | |
|---|---|
| HPMCP55 5% in acetone-ethanol | 1000 g |

After desiccation in a drier at 35°C for 14 hours, the spheroids were encapsulated by dosing a quantity equivalent to 5 and 10 mg / capsule of β-NADH, utilizing a Bonapace capsule machine, In-Cap model. The obtained capsules were tested to evaluate the in-vitro dissolution, thus obtaining the following results:

| | | |
|---|---|---|
| β-NADH mg content per capsule: | 5 | 10 |
| After 2 hours in HCl 0,1 N | 3% | |
| After 1 hour in pH 6,8 | 95% | |

### Example n° 8.5 - Enteric Coated formulation

1 Kg of spheroids obtained as described in example 8 was put in a Glatt fluid bed, Uniglatt model, and coated with the following suspension:

| | |
|---|---|
| Pregelatinised acetylated starch | 600 g |
| Water | 3400 g |
| Tot. | 4000 g |

### Example n° 8.6 - Enteric coated formulation

1 Kg of spheroids obtained as described in example 8 was put in a Glatt fluid bed, Uniglatt model, and coated with the following suspension:

| | |
|---|---|
| Pregelatinised acetylated starch | 500 g |
| Talc | 25 g |
| Shellac | 25 g |
| Ammonium carbonate | 2,5 g |
| Glycerol | 50 g |
| Water | 5580 g |
| Tot. | 6182,5 g |

The spheroids of examples 8.5 and 8.6 were tested to verify enteric coating and enteric dissolution, thus obtaining results in compliance with the preceding examples.

## Claims

1. Formulations for the oral administration of NADH in multiparticulate enteric coated form.

2. Formulations according to claim 1 in prolonged release form.

3. Formulations according to claim 1 dosed either into gelatine capsules or sachets or dispensers.

4. Formulations according to the preceding claims, containing NADH or NADPH or salts or physiologically acceptable derivatives of the same.

5. Formulations according to claim 1 obtained by layering NADH on inert cores, eventually with binder excipients like polyvinylpyrrolidone or polyethyleneglycol or derivatives, stabilizers like tocopherol and/or ascorbic palminate, and other excipients derived from the established pharmaceutical techniques.

6. Formulations according to claim 1 in which the cores containing NADH are prepared by extrusion and / or spheronization processes, utilizing binders like polyvinylpyrrolidone or polyethyleneglycol or derivatives, plasticizers like microcrystalline cellulose or starch or derivatives, and other excipients derived from the established pharmaceutical techniques.

7. Formulations according to claims 5 and 6 coated with enteric coating agents like hydroxypropylmethylcellulosephtalate, celluloseacetatephtalate, hydroxypropylmethylcellulosesuccinate, polymers of acrylic and metacrylic acid, other derivatives from the pregelatinised acetylated starch cellulose, shellac, polyvinylpyrrolidone, eventually added with plasticizer agents and/or colorants and every mixture in various proportion of the same.

8. Formulations according to claims 5 and 6 coated with substances suitable to modify the release of NADH, like polymers of the acrylic or metacrylic acid, ethylcellulose or other derivatives of the cellulose, shellac, paraffin, fat acids or mixtures in various proportions of such components eventually added with plasticizer agents and/or colorants subsequently coated to guarantee the enteric coating.

9. Formulations containing NADH according to what described in the examples.
